(19) Europäisches Patentamt
European Patent Office
Office européen des brevets

(11) Veröffentlichungsnummer: **0 051 792**
**A1**

# (12) EUROPÄISCHE PATENTANMELDUNG

(21) Anmeldenummer: **81108966.3**

(22) Anmeldetag: **27.10.81**

(51) Int. Cl.³: **C 07 C 131/105**, C 07 C 83/10, C 07 C 119/18, C 07 C 149/24, C 07 D 273/00, A 01 N 37/22, A 01 N 37/36, A 01 N 43/88

(30) Priorität: **08.11.80 DE 3042243**

(43) Veröffentlichungstag der Anmeldung: **19.05.82 Patentblatt 82/20**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Führer, Wolfgang, Dr., Wehrstrasse 28, D-5202 Hennef 1 (DE)**
Erfinder: **Stetter, Jörg, Dr., Gellertweg 4, D-5600 Wuppertal 1 (DE)**
Erfinder: **Eue, Ludwig, Dr., Paul-Klee-Strasse 36, D-5090 Leverkusen (DE)**
Erfinder: **Schmidt, Robert Rudolf, Dr., Im Waldwinkel 110, D-5060 Bergisch-Gladbach (DE)**

(54) **Hydroxamsäureester-Derivate, Verfahren zu ihrer Herstellung sowie ihre Verwendung als Herbizide.**

(57) Hydroxamsäureester-Derivate der Formel

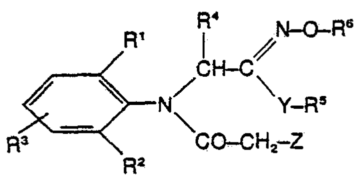

(I)

in welcher
Y für Sauerstoff oder Schwefel steht,
$R^1$ für Wasserstoff, Alkyl oder Alkoxy steht,
$R^2$ für Wasserstoff, Alkyl, Alkoxy oder Halogen steht,
$R^3$ für Wasserstoff, Alkyl, Alkoxy oder Halogen steht,
$R^4$ für Wasserstoff oder Alkyl steht,
$R^5$ für Alkyl, Alkenyl, Alkinyl oder Alkoxyalkyl steht,
$R^6$ für Wasserstoff, Alkyl, Alkenyl, Alkinyl oder Alkoxyalkyl steht oder
$R^4$ und $R^5$ gemeinsam für eine Alkylen-Kette stehen, oder
$R^5$ und $R^6$ gemeinsam für eine Alkylen-Kette stehen, sofern Y für Sauerstoff steht und
Z für Halogen steht,
ein Verfahren zu deren Herstellung und deren Verwendung als Herbizide.

N-Phenylglycin-Derivate der Formel

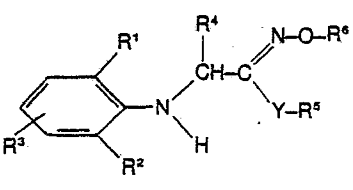

(II)

in welcher
Y, $R^1$, $R^2$, $R^3$, $R^4$, $R^5$ und $R^6$ die oben angegebenen Bedeutungen haben,
mehrere Verfahren zu deren Herstellung und deren Verwendung als Zwischenprodukte zur Synthese der Stoffe der Formel (I).

EP 0 051 792 A1

0051792

BAYER AKTIENGESELLSCHAFT 5090 Leverkusen, Bayerwerk
Zentralbereich
Patente, Marken und Lizenzen Dü/kl-c
Ia

# Hydroxamsäureester-Derivate, Verfahren zu ihrer Herstellung sowie ihre Verwendung als Herbizide

Die vorliegende Erfindung betrifft neue Hydroxamsäureester-Derivate, ein Verfahren zu ihrer Herstellung und ihre Verwendung als Herbizide.

Es ist bereits bekannt geworden, daß bestimmte Oximether herbizide Eigenschaften besitzen (vgl. DE-OS 28 47 827). So lassen sich beispielsweise 2,6-Diethyl-N-(1'-methyl-2'-ethoxyiminoethyl)-chloracetanilid, 2,6-Dimethyl-N-(1'-methyl-2'-methoxyimino-ethyl)-chloracetanilid und 2,6-Diethyl-N-(2'-propoxyimino-ethyl)-chloracetanilid zur Unkrautbekämpfung verwenden. Diese Verbindungen sind jedoch nicht immer ausreichend wirksam und in ihrer Selektivität nicht immer ganz befriedigend.

Es wurden nun neue Hydroxamsäureester-Derivate der Formel

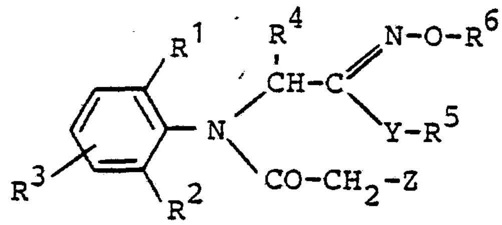

(I)

in welcher

Y für Sauerstoff oder Schwefel steht und

$R^1$ für Wasserstoff, Alkyl oder Alkoxy steht,

$R^2$ für Wasserstoff, Alkyl, Alkoxy oder Halogen steht,

$R^3$ für Wasserstoff, Alkyl, Alkoxy oder Halogen steht,

$R^4$ für Wasserstoff oder Alkyl steht,

$R^5$ für Alkyl, Alkenyl, Alkinyl oder Alkoxyalkyl steht,

$R^6$ für Wasserstoff, Alkyl, Alkenyl, Alkinyl oder Alkoxyalkyl steht oder

$R^4$ und $R^5$ gemeinsam für eine Alkylen-Kette stehen, oder

$R^5$ und $R^6$ gemeinsam für eine Alkylen-Kette stehen, sofern Y für Sauerstoff steht und

Z für Halogen steht,

gefunden.

Die Verbindungen der Formel (I) können in der syn- oder anti-Form vorliegen. Sie fallen in der Regel als Gemische beider Isomeren an.

Weiterhin wurde gefunden, daß man die Hydroxamsäureester-Derivate der Formel (I) erhält, wenn man N-Phenylglycin-Derivate der Formel

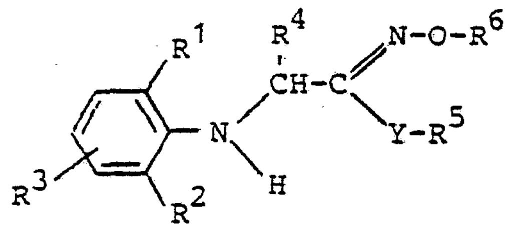

(II)

in welcher

$R^1$, $R^2$, $R^3$, $R^4$, $R^5$, $R^6$ und Y die obengenannte Bedeutung haben,

mit Halogenessigsäurechloriden oder -bromiden bzw. -anhydriden der Formeln

$$Z\text{-}CH_2\text{-}CO\text{-}Cl(Br) \qquad \text{(IIIa)}$$

bzw.

$$(Z\text{-}CH_2\text{-}CO)_2O \qquad \text{(IIIb)}$$

in welchen

Z die oben angegebene Bedeutung hat,

in Gegenwart eines Verdünnungsmittels und gegebenenfalls in Gegenwart eines Säurebindemittels umsetzt.

Schließlich wurde gefunden, daß die neuen Hydroxamsäureester-Derivate der Formel (I) starke herbizide, insbesondere auch selektiv-herbizide Eigenschaften besitzen.

Die erfindungsgemäßen Verbindungen der Formel (I) sind auch fungizid wirksam, insbesondere gegen Erreger der Oomyceten-Klasse.

Überraschenderweise zeigen die erfindungsgemäßen Hydroxamsäureester-Derivate der Formel (I) eine bessere herbizide Wirksamkeit als die aus dem Stand der Technik bekannten Verbindungen 2,6-Diethyl-N-(1'-methyl-2'-ethoxyimino-ethyl)-chloracetanilid, 2,6-Dimethyl-N-(1'-methyl-2'-methoxyimino-ethyl)-chloracetanilid und 2,6-Diethyl-N-(2'-propoxyimino-ethyl)-chloracetanilid, welches die konstitutionell ähnlichsten vorbekannten Wirkstoffe gleicher Wirkungsrichtung sind. Vor allem ist hervorzuheben, daß die erfindungsgemäßen Stoffe die genannten vorgeschriebenen Substanzen auch bezüglich ihrer selektiven herbiziden Wirksamkeit in wichtigen Kulturen übertreffen. Die erfindungsgemäßen Wirkstoffe stellen somit eine wertvolle Bereicherung der Technik dar.

Die erfindungsgemäßen Hydroxamsäureester-Derivate sind durch die Formel (I) allgemein definiert. In dieser Formel steht $R^1$ vorzugsweise für Wasserstoff, Alkyl mit 1 bis 4 Kohlenstoffatomen oder Alkoxy mit 1 bis 4 Koh-

lenstoffatomen. $R^2$ steht vorzugsweise für Wasserstoff, Alkyl mit 1 bis 4 Kohlenstoffatomen, Alkoxy mit 1 bis 4 Kohlenstoffatomen, Fluor, Chlor oder Brom. $R^3$ steht ebenfalls vorzugsweise für Wasserstoff, Alkyl mit 1 bis 4 Kohlenstoffatomen, Alkoxy mit 1 bis 4 Kohlenstoffatomen, Fluor, Chlor oder Brom. $R^4$ steht vorzugsweise für Wasserstoff oder Alkyl mit 1 bis 4 Kohlenstoffatomen. $R^5$ steht vorzugsweise für Alkyl mit 1 bis 4 Kohlenstoffatomen, Alkenyl mit bis zu 4 Kohlenstoffatomen, Alkinyl mit bis zu 4 Kohlenstoffatomen oder für Alkoxyalkyl mit 1 bis 4 Kohlenstoffatomen im Alkylteil und 1 bis 4 Kohlenstoffatomen im Alkoxyteil. $R^6$ steht vorzugsweise für Wasserstoff, Alkyl mit 1 bis 4 Kohlenstoffatomen, Alkenyl mit bis zu 4 Kohlenstoffatomen, Alkinyl mit bis zu 4 Kohlenstoffatomen oder für Alkoxyalkyl mit 1 bis 4 Kohlenstoffatomen im Alkylteil und 1 bis 4 Kohlenstoffatomen im Alkoxyteil. Außerdem stehen $R^4$ und $R^5$ auch gemeinsam für eine Alkylenkette mit vorzugsweise 1 bis 3 Kohlenstoffatomen. Weiterhin stehen auch $R^5$ und $R^6$ gemeinsam für eine Alkylenkette mit vorzugsweise 1 bis 3 Kohlenstoffatomen, sofern Y für Sauerstoff steht. Y steht für Sauerstoff oder Schwefel, und Z steht vorzugsweise für Chlor oder Brom.

Ganz besonders bevorzugt sind diejenigen Stoffe der Formel (I), in denen $R^1$ für Wasserstoff, Methyl, Ethyl, n-Propyl, Isopropyl, n-Butyl, sek.-Butyl, tert.-Butyl, Methoxy, Ethoxy oder Isopropoxy steht; $R^2$ für Wasserstoff, Methyl, Ethyl, n-Propyl, Isopropyl, n-Butyl, sek.-Butyl, tert.-Butyl, Methoxy, Ethoxy, Isopropoxy, Fluor oder Chlor steht; $R^3$ für Wasserstoff, Methyl,

Ethyl, n-Propyl, Isopropyl, n-Butyl, sek.-Butyl, tert.-Butyl, Methoxy, Ethoxy, Isopropoxy, Fluor oder Chlor steht; $R^4$ für Wasserstoff, Methyl oder Ethyl steht; $R^5$ für Methyl, Ethyl, n-Propyl, Isopropyl, n-Butyl, Allyl, Butenyl, Propargyl, Butinyl, Methoxymethyl, Methoxyethyl, Ethoxymethyl oder Ethoxyethyl steht; $R^6$ für Wasserstoff, Methyl, Ethyl, n-Propyl, Isopropyl, n-Butyl, Allyl, Butenyl, Propargyl, Butinyl, Methoxymethyl, Methoxyethyl, Ethoxymethyl oder Ethoxyethyl steht; oder $R^4$ und $R^5$ bzw. $R^5$ und $R^6$ gemeinsam für Methylen, Ethylen oder Propylen stehen; Y für Sauerstoff oder Schwefel steht und Z für Chlor oder Brom steht.

Verwendet man 0,0'-Dimethyl-N-(2,6-dimethylphenyl)-glycin-hydroxamsäure und Chloracetylchlorid als Ausgangsstoffe, so kann der Ablauf des erfindungsgemäßen Verfahrens durch das folgende Formelschema wiedergegeben werden:

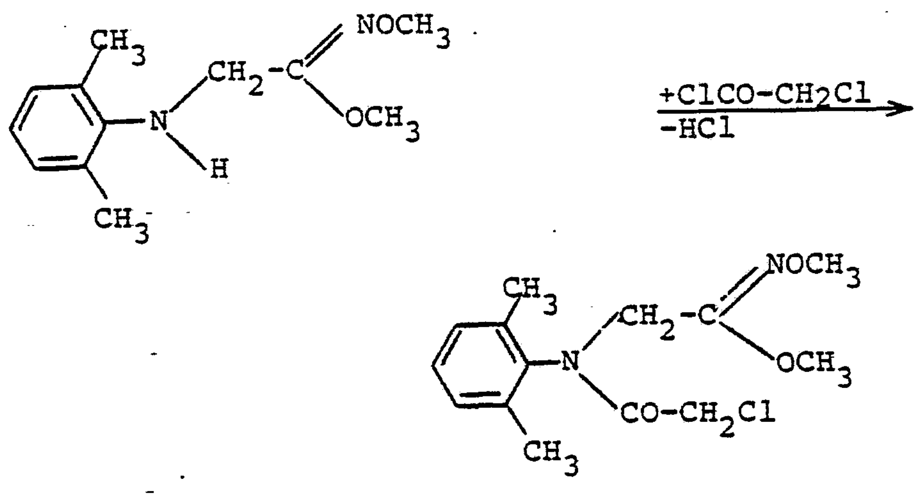

 0051792

Die bei der Durchführung des erfindungsgemäßen Verfahrens als Ausgangsstoffe benötigten N-Phenylglycin-Derivate sind durch die Formel (II) allgemein definiert. In dieser Formel haben $R^1$, $R^2$, $R^3$, $R^4$, $R^5$, $R^6$ und Y vorzugsweise diejenigen Bedeutungen, die bereits im Zusammenhang mit der Beschreibung der erfindungsgemäßen Stoffe vorzugsweise für diese Reste genannt wurden.

Die N-Phenylglycin-Derivate der Formel (II) sind bisher noch nicht bekannt; sie lassen sich jedoch in einfacher Weise herstellen. So erhält man

a) N-Phenylglycim-Derivate der Formel (II), indem man Anilin-Derivate der Formel

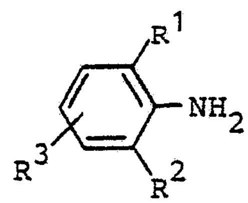

(IV)

in welcher

$R^1$, $R^2$ und $R^3$ die oben angegebene Bedeutung haben,

mit Hydroxamsäure-Derivaten der Formel

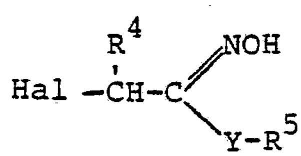

(V)

in welcher

$R^4$, $R^5$ und Y die oben angegebene Bedeutung haben und

Hal für Halogen, vorzugsweise für Chlor, Brom oder Jod steht,

in Gegenwart eines Verdünnungsmittels sowie gegebenenfalls in Gegenwart eines Säurebindemittels umsetzt und die dabei entstehenden N-Phenylglycin-Derivate der Formel

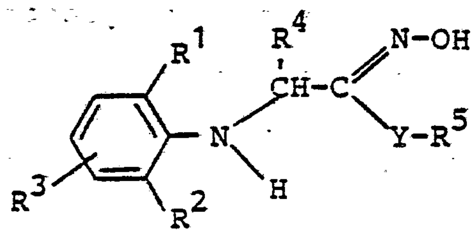

(IIa)

in welcher

$R^1$, $R^2$, $R^3$, $R^4$, $R^5$ und Y die oben angegebene Bedeutung haben,

gegebenenfalls anschließend mit Verbindungen der Formel

$$X-R^7 \quad \text{(VI)}$$

in welcher

$R^7$ für Alkyl, Alkenyl, Alkinyl oder Alkoxyalkyl steht und

X für Halogen oder ein Äquivalent eines Sulfat-Anions steht,

in Gegenwart eines Verdünnungsmittels sowie gegebenenfalls in Gegenwart eines Säurebindemittels umsetzt,

oder

b) N-Phenylglycin-Derivate der Formel (II), indem man Anilin-Derivate der Formel

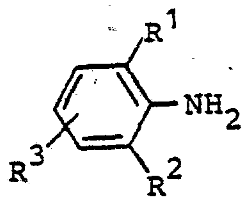

(IV)

in welcher

$R^1$, $R^2$ und $R^3$ die oben angegebene Bedeutung haben,

mit Hydroxamsäure-Derivaten der Formel

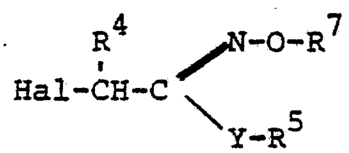

(VII)

in welcher

$R^4$, $R^5$, $R^7$, Hal und Y die oben angegebene Bedeutung haben,

in Gegenwart eines Verdünnungsmittels sowie gegebenenfalls in Gegenwart eines Säurebindemittels umsetzt,

oder

c) diejenigen N-Phenylglycin-Derivate der Formel (II), in denen Y für Sauerstoff steht und $R^5$ und $R^6$ gemeinsam für Alkylen stehen, indem man Anilin-Derivate der Formel

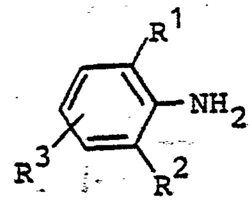

(IV)

in welcher

$R^1$, $R^2$ und $R^3$ die oben angegebene Bedeutung haben,

mit ringförmig verknüpften Alkylhydroxamsäureestern der Formel

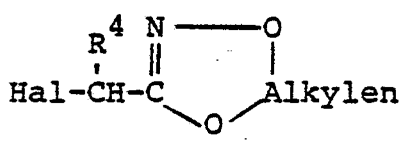

(VIII)

in welcher

$R^4$ und Hal die oben angegebene Bedeutung haben und
Alkylen für eine Alkylenkette steht,

in Gegenwart eines Verdünnungsmittels sowie gegebenenfalls in Gegenwart eines Säurebindemittels umsetzt.

Die bei den Verfahren (a) bis (c) als Ausgangsstoffe benötigten Aniline sind durch die Formel (IV) allgemein definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden.

Die Anilin-Derivate der Formel (IV) sind allgemein bekannte Verbindungen der organischen Chemie. Als Beispiele seien genannt:

Anilin; 2-Methylanilin; 2-Ethylanilin; 2-Isopropylanilin; 2-sek.-Butylanilin; 2-tert.-Butylanilin; 2,6-Dimethylanilin; 2,3-Dimethylanilin; 2,5-Dimethylanilin; 2,4-Dimethylanilin; 2,6-Diethylanilin; 2-Ethyl-6-methylanilin; 2,4,6-Trimethylanilin; 2-Ethyl-4,6-dimethylanilin; 2,6-Diethyl-4-methylanilin; 2,6-Diisopropyl-4-methylanilin; 2,3,6-Trimethylanilin; 2-Methyl-6-chloranilin; 2-tert.-Butyl-6-chloranilin; 2-Methoxy-6-methylanilin; 2,6-Dimethoxyanilin; 2-Methoxy-6-ethylanilin; 2,6-Diethoxyanilin.

Die bei den Verfahren (a) und (b) weiterhin als Ausgangskomponenten benötigten Hydroxamsäure-Derivate sind durch die Formeln (V) und (VII) allgemein definiert. In diesen Formeln haben $R^4$, $R^5$, $R^6$ und Y vorzugsweise diejenigen Bedeutungen, die bereits im Zusammenhang mit der Beschreibung der erfindungsgemäßen Stoffe der Formel (I) vorzugsweise für diese Reste genannt wurden. $R^7$ steht vorzugsweise für Alkyl mit 1 bis 4 Kohlenstoffatomen, Alkenyl mit bis zu 4 Kohlenstoffatomen, Alkinyl mit bis zu 4 Kohlenstoffatomen oder für Alkoxyalkyl mit 1 bis 4 Kohlenstoffatomen im Alkoxyteil und 1 bis 4 Kohlenstoffatomen im Alkylteil. Hal steht vorzugsweise für Chlor, Brom oder Jod.

Die Hydroxamsäure-Derivate der Formeln (V) und (VII) sind teilweise bekannt (vgl. DE-OS 1 937 454, GB-PS 1 275 806 und US-PS 3 644 387).

Die für das Verfahren (a) erforderlichen Hydroxamsäure-Derivate der Formel (V) lassen sich herstellen, indem man Iminoester der Formel

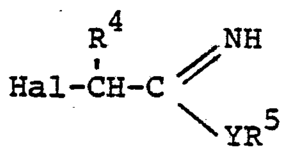

(IX)

in welcher

$R^4$, $R^5$, Y und Hal die oben angegebene Bedeutung haben,

in Gegenwart eines inerten organischen Verdünnungsmittels, wie z.B. Diethylether, mit wäßrigen Lösungen von Hydroxylamin-Hydrochlorid, das vorzugsweise in 50 bis 100 %igem Überschuß verwendet wird, bei Temperaturen zwischen -20°C und +20°C umsetzt.

Die Verbindungen der Formel (IX) sind in Form ihrer Salze bekannt oder lassen sich nach bekannten Methoden herstellen (vgl. DE-OS 2 746 057).

Die bei dem Verfahren (a) als Reaktionskomponenten benötigten Verbindungen sind durch die Formel (VI) allgemein definiert. In dieser Formel steht $R^7$ vorzugsweise für diejenigen Bedeutungen, die oben bereits für diesen Rest als vorzugsweise in Frage kommend genannt wurden. X steht vorzugsweise für Chlor, Brom, Jod oder ein Äquivalent eines Sulfat-Anions.

Die Verbindungen der Formel (VI) sind bekannt oder lassen sich nach bekannten Verfahren herstellen.

Die Hydroxamsäure-Derivate der Formel (VII) lassen sich herstellen, indem man Verbindungen der Formel

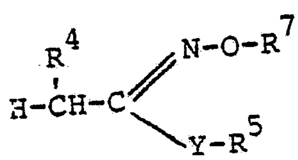

(X)

in welcher

$R^4$, $R^5$, $R^7$ und Y die oben angegebene Bedeutung haben,

in Gegenwart eines Verdünnungsmittels, wie zum Beispiel Tetrachlorkohlenstoff, mit Halogenierungsmitteln, wie

beispielsweise N-Bromsuccinimid, bei Temperaturen zwischen -20°C und +100°C halogeniert.

Die Verbindungen der Formel (X) unterscheiden sich von denen der Formel (VII) nur durch den Ersatz von Hal durch Wasserstoff. Sie können infolgedessen, soweit sie nicht ohnehin bekannt sind, in analoger Weise hergestellt werden wie die Stoffe der Formel (VII).

Die Hydroxamsäure-Derivate der Formel (VII) können auch, insbesondere für den Fall, daß $R^4$ und $R^5$ gemeinsam eine Alkylen-Brücke bilden, oder daß Y für Schwefel steht, hergestellt werden, indem man Hydroxy-Verbindungen der Formel

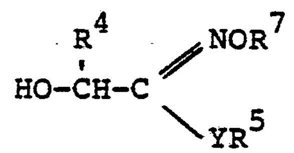

(XI)

in welcher

$R^4$, $R^5$, $R^7$ und Y die oben genannte Bedeutung haben,

in Gegenwart eines Verdünnungsmittels, wie zum Beispiel Petrolether, Cyclohexan, Toluol, gegebenenfalls in Anwesenheit eines säurebindenden Mittels, wie beispielsweise Pyridin oder Triethylamin, mit Halogenierungsmitteln, wie zum Beispiel Thionylchlorid, bei Temperaturen zwischen 0°C und 120°C, vorzugsweise zwischen 60°C und 90°C, umsetzt.

Die Verbindungen der Formel (XI) erhält man zweckmäßigerweise, indem man Ketone der Formel

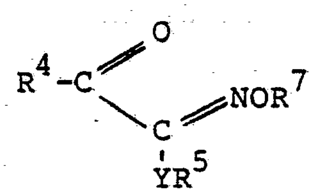

(XII)

in welcher

$R^4$, $R^5$, $R^7$ und Y die oben angegebene Bedeutung haben,

gegebenenfalls in Gegenwart eines Verdünnungsmittels reduziert. Vorzugsweise werden komplexe Hydride, wie zum Beispiel Natriumboranat, und polare Lösungsmittel, wie Wasser, Methanol, Ethanol, Isopropanol oder Tetrahydrofuran bzw. Gemische mehrerer dieser Komponenten bei Temperaturen zwischen 0°C und 80°C eingesetzt.

Die Verbindungen der Formel (XII) sind teilweise bekannt. Sie werden erhalten durch Umsatz der allgemein bekannten Ketone der Formel

$$R^4-\overset{O}{\overset{\parallel}{C}}-CH_2-YR^5 \qquad (XIII)$$

in welcher

$R^4$, $R^5$ und Y die oben genannte Bedeutung haben,

mit Nitrosierungsmitteln, wie zum Beispiel Isoamylnitrit, vorzugsweise in Gegenwart äquimolarer Mengen von Alkalialkoholat und in Gegenwart eines Verdünnungsmittels wie Alkohol und anschließende Alkylierung der dabei entstehenden Derivate der Formel

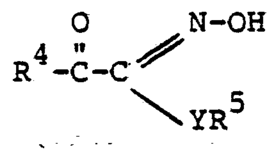

(XIIIa)

in welcher

$R^4$, $R^5$ und Y die oben angegebene Bedeutung haben,

mit Alkylierungsmitteln der Formel (VI) in der oben beschriebenen Weise.

Die bei dem Verfahren (c) als Reaktionskomponenten benötigten ringförmig verknüpften Alkylhydroxamsäureester sind durch die Formel (VIII) allgemein definiert. In dieser Formel hat $R^4$ vorzugsweise diejenigen Bedeutungen, die bereits im Zusammenhang mit der Beschreibung der erfindungsgemäßen Stoffe der Formel (I) vorzugsweise für diesen Rest genannt wurden. Alkylen steht vorzugsweise für eine Alkylenkette mit 1 bis 3 Kohlenstoffatomen. Hal steht vorzugsweise für Chlor, Brom oder Jod.

Die ringförmig verknüpften Alkylhydroxamsäureester der Formel (VIII) sind bisher noch nicht bekannt. Sie lassen sich jedoch herstellen, indem man Hydroxamsäuren der Formel

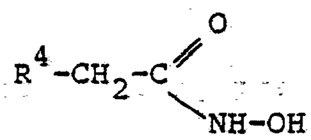 (XIV)

in welcher

$R^4$ die oben angegebene Bedeutung hat,

mit Dihalogenalkylenen der Formel

Hal-Alkylen-Hal (XV)

in welcher

Alkylen die oben angegebene Bedeutung hat und

Hal für Chlor, Brom oder Jod steht,

in Gegenwart eines Verdünnungsmittels, wie z.B. Wasser Methanol, Ethanol, Acetonitril, Dimethylformamid o.a. sowie in Gegenwart eines Säurebindemittels, wie z.B. Kaliumcarbonat, bei Temperaturen zwischen 10°C und 50°C umsetzt und die dabei anfallenden Verbindungen der Formel

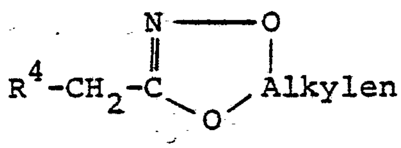

(XVI)

in welcher

$R^4$ und Alkylen die oben angegebene Bedeutung haben,

in Gegenwart eines Verdünnungsmittels, wie z.B. Tetrachlorkohlenstoff, mit Halogenierungsmitteln, wie zum Beispiel N-Bromsuccinimid bei Temperaturen zwischen 0°C und 100°C halogeniert.

Die Verbindungen der Formeln (XIV) und (XV) sind bekannt.

Als Verdünnungsmittel können bei den Verfahren (a) bis (c) alle inerten organischen Solventien verwendet werden. Vorzugsweise in Frage kommen Ether, wie Diethylether, Dioxan oder Tetrahydrofuran, ferner aromatische Kohlenwasserstoffe, wie Benzol, Toluol und Xylol, weiterhin Ketone, wie Aceton oder Butanon, Nitrile, wie Acetonitril, Amide, wie Dimethylformamid, sowie Alkohole, wie Methanol oder Ethanol, und Ester, beispielsweise Essigsäureethylester.

Die Verfahren (a) bis (c) werden vorzugsweise in Gegenwart eines Säurebindemittels durchgeführt. Als solche können alle üblichen Säureakzeptoren verwendet werden. Hierzu gehören vorzugsweise organische Basen, wie tertiäre Amine, beispielsweise Triethylamin und Pyridin, und ferner anorganische Basen, wie Alkalihydroxide und Alkalicarbonate.

Die Reaktionstemperaturen können bei den Verfahren (a) bis (c) in einem größeren Bereich variiert werden. Im allgemeinen arbeitet man bei Temperaturen zwischen 20°C und 160°C, vorzugsweise zwischen 60°C und 120°C.

Bei der Durchführung der Verfahren (a) bis (c) setzt man die Reaktionskomponenten vorzugsweise in äquimolaren Mengen ein. Es ist jedoch auch möglich, eine der Komponenten, vorzugsweise das Anilin-Derivat der Formel (IV), in einem Überschuß einzusetzen. Die Aufarbeitung und die Isolierung der Reaktionsprodukte erfolgt nach üblichen Methoden.

Die außerdem für das erfindungsgemäße Verfahren als Ausgangsstoffe zu verwendenden Halogenessigsäurechloride und -bromide bzw. -anhydride sind durch die Formeln (IIIa) und (IIIb) allgemein definiert. In diesen Formeln steht Z vorzugsweise für Chlor, Brom und Jod.

Die Halogenessigsäurechloride und -bromide bzw. -anhydride der Formeln (IIIa) und (IIIb) sind allgemin bekannte Verbindungen der organischen Chemie. Als Beispiele seien genannt: Chloracetylchlorid, Bromacetylchlorid, Jodacetylchlorid und die entsprechenden Bromide sowie Anhydride.

Als Verdünnungsmittel kommen für das erfindungsgemäße Verfahren alle inerten organischen Lösungsmittel in Frage. Hierzu gehören vorzugsweise Ketone, wie Diethylketon, insbesondere Aceton und Methylethylketon; Nitrile, wie Propionitril, insbesondere Acetonitril; Ether wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Das erfindungsgemäße Verfahren kann in Gegenwart von Säurebindern (Halogenwasserstoff-Akzeptoren) durchgeführt werden. Als solche können alle üblichen Säurebindemittel verwendet werden. Hierzu gehören vorzugsweise organische Basen, wie tertiäre Amine, beispielsweise Triethylamin, oder Pyridin, ferner anorganische Basen, wie beispielsweise Alkalihydroxide und Alkalicarbonate.

Die Reaktionstemperaturen können bei der Durchführung des erfindungsgemäßen Verfahrens in einem größeren Bereich variiert werden. Im allgemeinen arbeitet man zwischen 0°C und 120°C, vorzugsweise zwischen 20 und 100°C.

Bei der Durchführung des erfindungsgemäßen Verfahrens setzt man vorzugsweise auf 1 Mol der Verbindungen der Formel (II) 1 bis 1,5 Mol Halogenacetylierungsmittel und 1 bis 1,5 Mol Säurebinder ein. Die Isolierung der Verbindungen der Formel (I) erfolgt in üblicher Weise.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten wachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen insbesondere neben einer sehr guten Wirkung gegen breitblättrige Unkräuter auch eine gute Wirkung gegen Gräser. Ein selektiver Einsatz der erfindungsgemäßen Wirkstoffe ist möglich, vorzugsweise in Baumwolle, Sojabohnen und

Rüben. Darüber hinaus besitzen die erfindungsgemäßen Stoffe zum Teil auch eine fungizide Wirksamkeit.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können als solche oder in ihren Formulierungen auch in Mischung mit bekannten Herbiziden zur Unkraubekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die erfindungsgemäßen Wirkstoffe zeigen bei Nachauflaufanwendung wachstumsregulierende Eigenschaften.

Herstellungsbeispiele

Beispiel 1

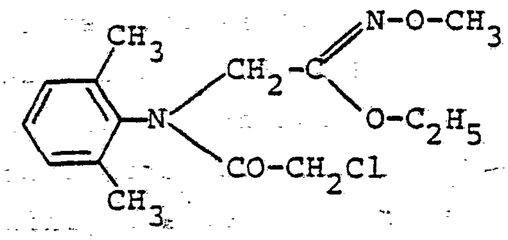

10 g (0,042 Mol) Ethyl-N-(2,6-dimethylphenyl)-glycin-hydroxamsäure-methylester werden in 50 ml Dichlormethan und 3,4 g (0,042 Mol) Pyridin auf 0°C gekühlt, tropfenweise mit 4,7 g (0,042 Mol) Chloracetylchlorid versetzt und bei Raumtemperatur über Nacht stehengelassen. Zur Aufarbeitung versetzt man das Reaktionsgemisch mit Wasser und trennt die organische Phase ab. Letztere wird dreimal mit Wasser gewaschen, über Natriumsulfat getrocknet und anschließend unter vermindertem Druck, zuletzt im Hochvakuum eingeengt. Das verbleibende Öl kristallisiert nach längerem Stehen und ergibt nach dem Verreiben mit Diisopropylether 4,7 g (35,5 % d.Th.) an farblosen Kristallen vom Schmelzpunkt 73-76°C.

Herstellung des Ausgangsstoffes:

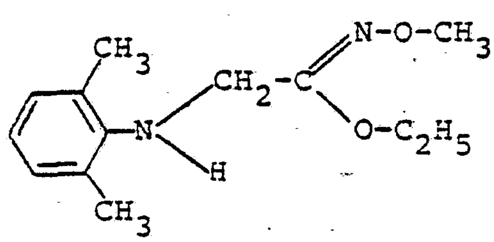

(II-1)

36,3 g (0,3 Mol) 2,6-Xylidin, 21 g (0,15 Mol) feingemahlenes Kaliumcarbonat und 40 ml Acetonitril werden unter Stickstoff bei Rückflußtemperatur gerührt, wobei man 20 g (0,15 Mol) Ethylchloracethydroxamsäure, gelöst in 40 ml Acetonitril, innerhalb von 2 Stunden zutropft. Nach weiteren 5 Stunden bei gleicher Temperatur wird der anorganische Rückstand abfiltriert, das Lösungsmittel entfernt und überschüssiges Xylidin im Hochvakuum bei maximal 90°C entfernt.

Nach Auflösen des Rückstandes in 100 ml Dichlormethan und Zugabe von 0,5 g Triethylbenzylammoniumchlorid wird mit 5,8 g Natriumhydroxid (0,14 Mol), gelöst in 20 ml Wasser, überschichtet, bei Raumtemperatur unter kräftigem Rühren mit 18,3 g (0,14 Mol) Dimethylsulfat tropfenweise versetzt und 9 Stunden nachgerührt. Die organische Phase wird abgetrennt, danach zweimal mit Wasser gewaschen und nach Trocknen über Natriumsulfat im Vakuum destilliert. In der Fraktion, die bei 95-105°C /0,6 mbar übergeht, erhält man 11,4 g (33,2 % d.Th.) des Stoffes der Formel (II-1) in Form eines farblosen Öles.

Beispiel 2

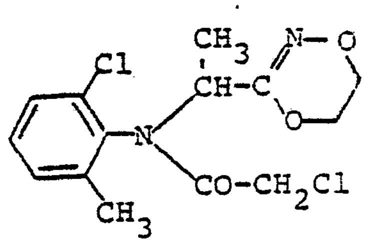

(I-2)

5 g (0,02 Mol) 3-α-(2-Chlor-6-methyl-anilino)-ethyl-1,4-diox-2-azin, 50 ml Dichlormethan und 3,2 g Pyridin (0,04 Mol) werden auf 0°C gekühlt, tropfenweise mit 3,3 g (0,03 Mol) Chloracetylchlorid versetzt und bei Raumtemperatur über Nacht stehengelassen. Zur Aufarbeitung versetzt man das Reaktionsgemisch mit Wasser und trennt die organische Phase ab. Nach dreimaligem Waschen der organischen Phase mit Wasser und Trocknen über Natriumsulfat zieht man das Reaktionsprodukt auf 30 g Kieselgel auf und eluiert mit jeweils 200 ml Cyclohexan/Aceton 100 : 1, 20 : 1, 10 : 1 und 2 : 1. Nach Abdampfen des Lösungsmittels erhält man in der Hauptfraktion 3,8 g (61,4 % d.Th.) des Stoffes der Formel (I-2) in Form eines hochviskosen Öles, das im Infrarotspektrum eine Anilidbande bei 1660 $cm^{-1}$ und eine C=N-Absorption bei 1620 $cm^{-1}$ zeigt. Dünnschichtchromatographisch ergibt sich im Laufmittel Petrolether/Essigester 5 : 2 ein $R_F$-Wert von 0,5.

Herstellung des Ausgangsstoffes:

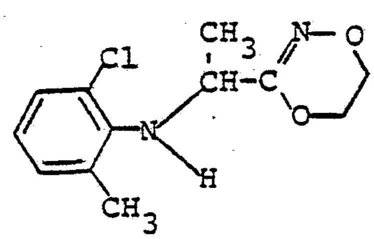

(II-2)

35 g (0,25 Mol) 2-Chlor-6-methylanilin, 14 g (o,1 Mol) feingemahlenes Kaliumcarbonat und 19 g (0,1 Mol) 3-(α-Bromethyl)-1,4-diox-2-azin werden unter Stickstoff 18 Stunden bei 140°C gerührt. Das Reaktionsgemisch wird dann in 100 ml Dichlormethan aufgenommen, filtriert, eingeengt und im Vakuum destilliert. Beim Siedepunkt von 138-141°C/0,3 mbar erhält man 16,3 g (65,3 % d.Th.) des Stoffes der Formel (II-2) in Form eines farblosen Öles.

Herstellung des Vorproduktes:

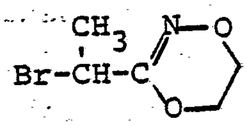

(XVI-1)

Die heiß hergestellten Lösungen von 139 g (2 Mol) Hydroxylamin-Hydrochlorid in 800 ml Methanol und 224 g (4 Mol) Kaliumhydroxid in 800 ml Methanol werden bei maximal 40°C vereinigt und sofort unter Rühren mit 176 g (2 Mol) Propionsäuremethylester versetzt. Nach Stehen über Nacht bei Raumtemperatur unter Luftausschluß filtriert man, versetzt die Mutterlauge mit 276 g (2 Mol) wasserfreiem Kaliumcarbonat, erwärmt auf 40°C und tropft 376 g (2 Mol) 1,2-Dibromethan ein. Nach 2,5-tägigem Nachrühren bei gleicher Temperatur wird vom Salz abfiltriert und die Mutterlauge destilliert. Beim Siedepunkt 73-76°C/16 mbar erhält man 86,8 g (37,8 % d.Th.) 3-Ethyl-1,4-diox-2-azin in Form eines farblosen viskosen Öles mit einem Brechungsindex $n_D^{20}$ = 1,4520.

0051792

Man löst 11,5 g (0,1 Mol) dieses 3-Ethyl-1,4-diox-2-azins in 100 ml Tetrachlorkohlenstoff, gibt 18 g (0,1 Mol) N-Bromsuccinimid zu und belichtet und erhitzt 1,5 Stunden unter Rückfluß. Nach Filtration und Destillation erhält man bei 95-100°C/0,13 mbar 15 g (77 % d.Th.) des Stoffes der Formel (XVI-1) in Form eines farblosen Öles mit dem Brechungsindex $n_D^{20}$= 1,5086.

Beispiel 3

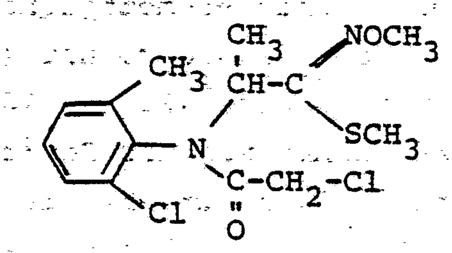

(I-3)

2,4 g (0,009 Mol) 1-Thiomethyl-1-methoximino-2-(2'-Chlor-6'-methyl-anilino)-propan, gelöst in 50 ml Dichlormethan und 1,1 g (0,014 Mol) Pyridin, werden auf 0°C gekühlt und tropfenweise mit 1,4 g (0,012 Mol) Chloracetylchlorid versetzt. Nach dem Stehen über Nacht wird 30 Minuten unter Rückfluß erhitzt. Danach wird das Reaktionsgemisch mit Wasser versetzt, und die organische Phase abgetrennt. Man wäscht die organische Phase zweimal mit Wasser, trocknet über Natriumsulfat und engt unter vermindertem Druck ein. Man erhält auf diese Weise 2,3 g (73,2 % d. Th.) an der Verbindung der Formel (I-3) in Form eines viskosen Öles mit einem Brechungsindex $n_D^{20}$= 1,5608.

 

Herstellung des Ausgangsstoffes:

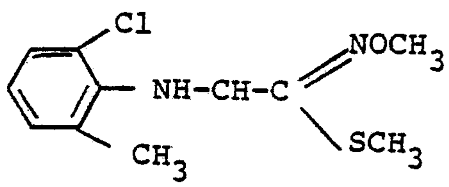

(II-3)

20 g (0,12 Mol) 1-Methoximino-1-thiomethyl-2-chlor-propan, 35 g (0,25 Mol) 2-Chlor-6-methylanilin, 20 g (0,14 Mol) feingepulvertes Kaliumcarbonat (wasserfrei) und 2 g Natriumjodid werden unter Rühren 10 Stunden auf 120°C erhitzt. Dann nimmt man das Reaktionsgemisch in 100 ml Dichlormethan auf, filtriert die anorganischen Bestandteile ab und destilliert das nach dem Entfernen des Lösungsmittels verbleibende Produkt im Hochvakuum.

Man erhält 5 g (15,3 % d.Th.) der Verbindung der Formel (II-3) in Form eines farblosen Öles mit einem Siedepunkt von 140°C/0,15 mbar und einem Brechungsindex $n_D^{20}$= 1,5703.

Herstellung der Vorstufe:

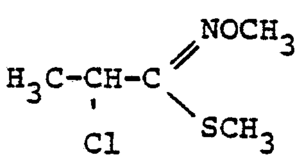

(VII-1)

a) 1-Oximino-1-thiomethyl-2-oxo-propan:

Zu einer Lösung von 30,5 g (1,33 Gat) Natrium in 1 l absolutem Alkohol werden 135 g (1,15 Mol) Isoamylnitrit gegeben; dann werden bei 0°C 125 g (1,2 Mol) Methylmercaptoaceton eingetropft. Die Mischung wird 4 Stunden bei Raumtemperatur nachgerührt. Danach dampft man das Reaktionsgemisch auf das halbe Volumen ein, setzt 500 ml Wasser zu, schüttelt die wäßrige Lösung dreimal mit Petrolether und einmal mit Chloroform aus, säuert dann mit konzentrierter Salzsäure auf pH 5 bis 6 an, extrahiert das Reaktionsprodukt mit dreimal je 100 ml Chloroform, trocknet über Natriumsulfat und dampft das Lösungsmittel ab. Man erhält 116,5 g (76,2 % d.Th.) von 1-Oximino-1-thiomethyl-2-oxo-propan in Form eines hellen Öles vom Brechungsindex $n_D^{20}$ = 1,5168.

b) 1-Methoximino-1-thiomethyl-2-oxo-propan:

Das Öl, dessen Herstellung unter a) beschrieben ist, wird in eine Lösung von 49 g (0,88 Mol) Kaliumhydroxid in 1 l Methanol eingetragen und 30 Minuten bei Raumtemperatur gerührt. Anschließend werden 121,4 g (0,96 Mol) Dimethylsulfat zugetropft. Danach erhitzt man 30 Minuten unter Rückfluß. Nach dem Verdünnen des Reaktionsgemisches mit Wasser auf das doppelte Volumen wird mit Kalilauge alkalisiert, dreimal mit Chloroform extrahiert, die organische Phase abgetrennt und mit Aktivkohle und Natriumsulfat behandelt. Nach dem Eindampfen verbleiben 65,8 g (51,2 % d.Th.) an 1-Methoximino-1-thiomethyl-2-oxo-propan in Form eines rötlichen Öles mit dem Brechungsindex $n_D^{20}$= 1,5023.

c) 1-Methoximino-1-thiomethyl-2-hydroxy-propan:

65 g (0,44 Mol) 1-Methoximino-1-thiomethyl-2-oxo-propan werden in 200 ml Alkohol gelöst und portionsweise mit insgesamt 25 g (0,66 Mol) Natriumboranat versetzt, wobei sich das Gemisch erwärmt. Man rührt eine Stunde nach, engt dann im Vakuum ein, nimmt in 200 ml Wasser auf und extrahiert fünfmal mit je 50 ml Chloroform. Man trocknet über Natriumsulfat und erhält nach dem Eindampfen durch Destillation des verbleibenden Rückstandes 53 g (80,4 % d. Th.) an 1-Oximino-1-thiomethyl-2-oxo-propan in Form eines viskosen, hellen Öles mit einem Brechungsindex von $n_D^{20}$= 1,5129 und einem Siedepunkt von 100-110°C bei 0,45 mbar.

d) 1-Methoximino-1-thiomethyl-2-chlor-propan:

46 g (0,31 Mol) 1-Methoximino-1-thiomethyl-2-hydroxy-propan werden in 300 ml Cyclohexan und 26,9 g (0,34 Mol) Pyridin unter Rückfluß und Feuchtigkeitsausschluß erhitzt und tropfenweise mit 40,3 g (0,34 Mol) Thionylchlorid versetzt. Man erhält die Temperatur bis zum Ende der Gasentwicklung konstant, versetzt das Reaktionsgemisch dann mit Wasser und trennt die Phasen.

Die organische Phase wird mit Wasser gewaschen, unter vermindertem Druck eingeengt, und der verbleibende Rückstand wird destilliert. Man erhält 32,8 g (63,4 % d.Th.) an 1-Methoximino-1-thiomethyl-2-chlor-propan mit einem Brechungsindex von $n_D^{20}$ = 1,5115 und einem Siedepunkt von 120-125°C bei 0,35 mbar.

Nach den beschriebenen Methoden werden auch die in der folgenden Tabelle 1 aufgeführten Stoffe der Formel (I) hergestellt:

Tabelle 1

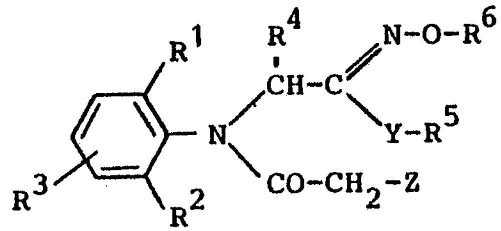

(I)

| Beispiel-Nr. | $R^1$ | $R^2$ | $R^3$ | $R^4$ | $R^5$ | $R^6$ | Y | Z | Schmelzp. [°C] bzw. Brechungsindex [$n_{20}^D$] |
|---|---|---|---|---|---|---|---|---|---|
| 4 | $CH_3$ | $CH_3$ | H | H | $CH_3$ | $CH_3$ | O | Cl | 1,5331 |
| 5 | $CH_3$ | $CH_3$ | H | H | $CH_3$ | $C_2H_5$ | O | Cl | 1,5319 |
| 6 | $CH_3$ | $CH_3$ | H | H | $CH_3$ | $-CH_2-CH=CH_2$ | O | Cl | 1,5238 |
| 7 | $CH_3$ | $CH_3$ | H | H | $C_2H_5$ | $C_2H_5$ | O | Cl | 59 |
| 8 | $CH_3$ | $CH_3$ | H | H | $n-C_3H_7$ | $-CH_2-CH=CH_2$ | O | Cl | 1,5191 |
| 9 | $CH_3$ | $CH_3$ | H | H | $n-C_3H_7$ | $C_2H_5$ | O | Cl | 1,5190 |
| 10 | $CH_3$ | $CH_3$ | H | H | $n-C_3H_7$ | $CH_3$ | O | Cl | 1,5221 |
| 11 | $CH_3$ | $C_2H_5$ | H | H | $C_2H_5$ | $CH_3$ | O | Cl | 1,5169 |
| 12 | $CH_3$ | $C_2H_5$ | H | H | $C_2H_5$ | $-CH_2-CH=CH_2$ | O | Cl | 1,5270 |
| 13 | $CH_3$ | $C_2H_5$ | H | H | $n-C_3H_7$ | $CH_3$ | O | Cl | 1,5240 |
| 14 | $CH_3$ | $C_2H_5$ | H | H | $n-C_3H_7$ | $C_2H_5$ | O | Cl | 1,5180 |
| 15 | $CH_3$ | $C_2H_5$ | H | H | $n-C_3H_7$ | $-CH_2-CH=CH_2$ | O | Cl | 1,5199 |

0051792

Tabelle 1 (Fortsetzung)

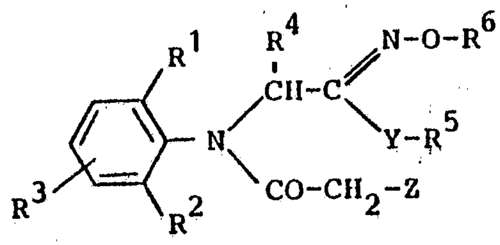

(I)

| Beispiel-Nr. | $R^1$ | $R^2$ | $R^3$ | $R^4$ | $R^5$ | $R^6$ | Y | Z | Schmelzp. [°C] bzw. Brechungsindex [$n_{20}^{D}$] |
|---|---|---|---|---|---|---|---|---|---|
| 16 | $CH_3$ | H | 3-$CH_3$ | H | $CH_3$ | $-CH_2-CH=CH_2$ | O | Cl | 1,5341 |
| 17 | $CH_3$ | H | 3-$CH_3$ | H | n-$C_3H_7$ | $CH_3$ | O | Cl | 1,5261 |
| 18 | $CH_3$ | H | 3-$CH_3$ | H | n-$C_3H_7$ | $-CH_2-CH=CH_2$ | O | Cl | 1,5268 |
| 19 | $C_2H_5$ | $C_2H_5$ | H | H | $CH_3$ | $-CH_2-CH=CH_2$ | O | Cl | 1,5288 |
| 20 | $C_2H_5$ | $C_2H_5$ | H | H | $C_2H_5$ | $-CH_2-CH=CH_2$ | O | Cl | 1,5250 |
| 21 | $C_2H_5$ | $C_2H_5$ | H | H | n-$C_3H_7$ | $CH_3$ | O | Cl | 1,5218 |
| 22 | $C_2H_5$ | $C_2H_5$ | H | H | n-$C_3H_7$ | $-CH_2-CH=CH_2$ | O | Cl | 1,5150 |
| 23 | $C_2H_5$ | $C_2H_5$ | H | H | n-$C_3H_7$ | $C_2H_5$ | O | Cl | 1,5179 |
| 24 | $CH_3$ | Cl | H | H | $C_2H_5$ | $CH_3$ | O | Cl | 67 |
| 25 | $CH_3$ | H | H | H | $CH_3$ | $CH_3$ | O | Cl | 1,5379 |
| 26 | $OCH_3$ | H | H | H | $CH_3$ | $CH_3$ | O | Cl | 1,5377 |

Tabelle 1 (Fortsetzung)

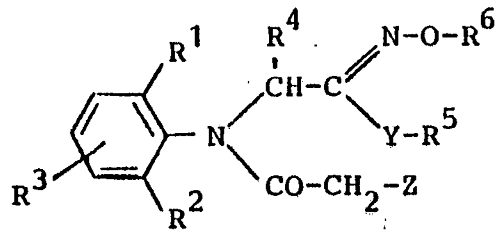

(I)

| Beispiel-Nr. | $R^1$ | $R^2$ | $R^3$ | $R^4$ | $R^5$ | $R^6$ | Y | Z | Schmelzp. [°C] bzw. Brechungsindex [$n_{20}^{D}$] |
|---|---|---|---|---|---|---|---|---|---|
| 27 | H | Cl | H | H | $C_2H_5$ | $CH_3$ | O | Cl | 1,5338 |
| 28 | $CH_3$ | $CH_3$ | H | $CH_3$ | $CH_3$ | $CH_3$ | O | Cl | 1,5320 |
| 29 | $CH_3$ | $C_2H_5$ | H | $CH_3$ | $CH_3$ | $CH_3$ | O | Cl | 1,5285 |
| 30 | $CH_3$ | Cl | H | $CH_3$ | $CH_3$ | $CH_3$ | O | Cl | 130-135; 1,5349 |
| 31 | $CH_3$ | $CH_3$ | H | $CH_3$ | $-CH_2-CH_2-$ | | O | Cl | hochviskoses Öl IR: 1660 $cm^{-1}$ C=O, 1620 $cm^{-1}$ C=N |
| 32 | $CH_3$ | Cl | H | $CH_3$ | $CH_3$ | $C_2H_5$ | S | Cl | 1,5590 |
| 33 | $CH_3$ | $CH_3$ | H | $CH_3$ | $CH_3$ | $C_2H_5$ | S | Cl | 1,5518 |
| 34 | $CH_3$ | $CH_3$ | H | H | $n-C_3H_7$ | $CH_2-C\equiv CH$ | O | Cl | 1,5308 |
| 35 | $CH_3$ | $C_2H_5$ | H | H | $n-C_3H_7$ | $CH_2-C\equiv CH$ | O | Cl | 1,5279 |
| 36 | $C_2H_5$ | $C_2H_5$ | H | H | $n-C_3H_7$ | $CH_2-C\equiv CH$ | O | Cl | 1,5300 |
| 37 | $CH_3$ | $CH_3$ | H | $CH_3$ | $C_2H_5$ | $C_2H_5$ | O | Cl | 1,5335 |

Nach den vorstehend beschriebenen Methoden werden die in der folgenden Tabelle 2 formelmäßig aufgeführten N-Phenylglycin-Derivate hergestellt.

Tabelle 2

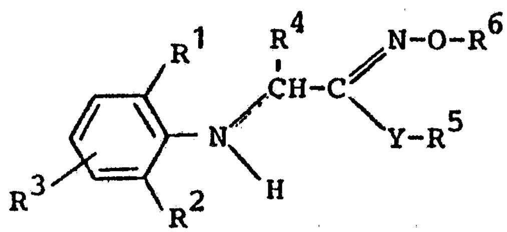

(II)

| Beispiel-Nr. | $R^1$ | $R^2$ | $R^3$ | $R^4$ | $R^5$ | $R^6$ | Y | Schmelzpunkt [°C] Siedepunkt [°C/mbar] bzw. Brechungsindex [$n_{20}^D$] |
|---|---|---|---|---|---|---|---|---|
| II-4 | $CH_3$ | $CH_3$ | H | H | $CH_3$ | H | O | viskoses Öl, nicht destillierbar |
| II-5 | $CH_3$ | $CH_3$ | H | H | $C_2H_5$ | H | O | " |
| II-6 | $CH_3$ | $CH_3$ | H | H | $n-C_3H_7$ | H | O | " |
| II-7 | $CH_3$ | $C_2H_5$ | H | H | $C_2H_5$ | H | O | " |
| II-8 | $CH_3$ | $C_2H_5$ | H | H | $n-C_3H_7$ | H | O | " |
| II-9 | $C_2H_5$ | $C_2H_5$ | H | H | $CH_3$ | H | O | " |
| II-10 | $C_2H_5$ | $C_2H_5$ | H | H | $C_2H_5$ | H | O | " |
| II-11 | $C_2H_5$ | $C_2H_5$ | H | H | $n-C_3H_7$ | H | O | " |
| II-12 | $CH_3$ | H | $3-CH_3$ | H | $CH_3$ | H | O | 122 |
| II-13 | $CH_3$ | H | $3-CH_3$ | H | $n-C_3H_7$ | H | O | viskoses Öl, nicht destillierbar |

Tabelle 2 (Fortsetzung)

| Beispiel-Nr. | $R^1$ | $R^2$ | $R^3$ | $R^4$ | $R^5$ | $R^6$ | Y | Siedepunkt [°C/mbar]; Schmelzpunkt [°C] bzw. Brechungsindex [$n_D^{20}$] |
|---|---|---|---|---|---|---|---|---|
| II-14 | $CH_3$ | Cl | H | H | $C_2H_5$ | H | O | viskoses Öl, nicht destillierbar |
| II-15 | $CH_3$ | H | H | H | $CH_3$ | H | O | 111 |
| II-16 | $OCH_3$ | H | H | H | $CH_3$ | H | O | 98 |
| II-17 | H | Cl | H | H | $C_2H_5$ | H | O | 88 - 92 |
| II-18 | $CH_3$ | $CH_3$ | H | H | $CH_3$ | $CH_3$ | O | 110/0,2 |
| II-19 | $CH_3$ | $CH_3$ | H | H | $CH_3$ | $C_2H_5$ | O | 130/0,2 |
| II-20 | $CH_3$ | $CH_3$ | H | H | $CH_3$ | $-CH_2-CH=CH_2$ | O | 140/1 |
| II-21 | $CH_3$ | $CH_3$ | H | H | $C_2H_5$ | $C_2H_5$ | O | 120/0,2 |
| II-22 | $CH_3$ | $CH_3$ | H | H | $n-C_3H_7$ | $CH_3$ | O | 120/0,7 |
| II-23 | $CH_3$ | $CH_3$ | H | H | $n-C_3H_7$ | $C_2H_5$ | O | 135/1 |
| II-24 | $CH_3$ | $CH_3$ | H | H | $n-C_3H_7$ | $-CH_2-CH=CH_2$ | O | 140/1 |
| II-25 | $CH_3$ | $C_2H_5$ | H | H | $C_2H_5$ | $CH_3$ | O | 115/0,4 |
| II-26 | $CH_3$ | $C_2H_5$ | H | H | $C_2H_5$ | $-CH_2-CH=CH_2$ | O | 140/0,9 |
| II-27 | $CH_3$ | $C_2H_5$ | H | H | $n-C_3H_7$ | $CH_3$ | O | 130/2 |

Tabelle 2 (Fortsetzung)

| Beispiel-Nr. | $R^1$ | $R^2$ | $R^3$ | $R^4$ | $R^5$ | $R^6$ | Y | Siedepunkt /°C/mbar/; Schmelzpunkt /°C/ bzw. Brechungsindex /$n_D^{20}$/ |
|---|---|---|---|---|---|---|---|---|
| II-28 | $CH_3$ | $C_2H_5$ | H | H | $n-C_3H_7$ | $C_2H_5$ | O | 130/0,1 |
| II-29 | $CH_3$ | $C_2H_5$ | H | H | $n-C_3H_7$ | $-CH_2-CH=CH_2$ | O | 150/0,1 |
| II-30 | $C_2H_5$ | $C_2H_5$ | H | H | $CH_3$ | $-CH_2-CH=CH_2$ | O | 1,5190 |
| II-31 | $C_2H_5$ | $C_2H_5$ | H | H | $C_2H_5$ | $-CH_2-CH=CH_2$ | O | 130/1 |
| II-32 | $C_2H_5$ | $C_2H_5$ | H | H | $n-C_3H_7$ | $CH_3$ | O | 115/1 |
| II-33 | $C_2H_5$ | $C_2H_5$ | H | H | $n-C_3H_7$ | $C_2H_5$ | O | 140/1 |
| II-34 | $C_2H_5$ | $C_2H_5$ | H | H | $n-C_3H_7$ | $-CH_2-CH=CH_2$ | O | 150/0,1 |
| II-35 | $CH_3$ | H | $3-CH_3$ | H | $CH_3$ | $-CH_2-CH=CH_2$ | O | 130/0,4 |
| II-36 | $CH_3$ | H | $3-CH_3$ | H | $n-C_3H_7$ | $CH_3$ | O | 140/0,1 |
| II-37 | $CH_3$ | H | $3-CH_3$ | H | $n-C_3H_7$ | $-CH_2-CH=CH_2$ | O | 150/1 |
| II-38 | $CH_3$ | Cl | H | H | $C_2H_5$ | $CH_3$ | O | 140/2; 1,5265 |
| II-39 | $CH_3$ | H | H | H | $CH_3$ | $CH_3$ | O | 130/0,3; 1,5406 |
| II-40 | $OCH_3$ | H | H | H | $CH_3$ | $CH_3$ | O | 130/0,8; 1,5451 |
| II-41 | H | Cl | H | H | $C_2H_5$ | $CH_3$ | O | 110/0,8; 1,5366 |
| II-42 | $CH_3$ | $CH_3$ | H | $CH_3$ | $CH_3$ | $CH_3$ | O | 115/0,3; 1,5180 |
| II-43 | $CH_3$ | $C_2H_5$ | H | $CH_3$ | $CH_3$ | $CH_3$ | O | 105/0,5; 1,5210 |
| II-44 | $CH_3$ | Cl | H | $CH_3$ | $CH_3$ | $CH_3$ | O | 130/0,2; 1,5510 |
| II-45 | $CH_3$ | $CH_3$ | H | $CH_3$ | $-CH_2-CH_2-$ | | O | 140/o,3; 1,5451 |
| II-46 | $CH_3$ | Cl | H | $CH_3$ | $CH_3$ | $C_2H_5$ | S | 160/0,15;1,5600 |
| II-47 | $CH_3$ | $CH_3$ | H | $CH_3$ | $CH_3$ | $C_2H_5$ | S | 140/0,5; 1,5580 |

Tabelle 2 (Fortsetzung)

| Beispiel-Nr. | $R^1$ | $R^2$ | $R^3$ | $R^4$ | $R^5$ | $R^6$ | Y | Siedepunkt /°C/mbar/; Schmelzpunkt /°C/ bzw. Brechungsindex /$n_D^{20}$/ |
|---|---|---|---|---|---|---|---|---|
| II-48 | $CH_3$ | $CH_3$ | H | H | n-$C_3H_7$ | $-CH_2-C{\equiv}CH$ | O | 150/0.3; 1,5238 |
| II-48 | $CH_3$ | $C_2H_5$ | H | H | n-$C_3H_7$ | $-CH_2-C{\equiv}CH$ | O | 180/0.4; 1,5183 |
| II-49 | $C_2H_5$ | $C_2H_5$ | H | H | n-$C_3H_7$ | $-CH_2-C{\equiv}CH$ | O | 160/0.4; 1,5164 |
| II-50 | $CH_3$ | $CH_3$ | H | $CH_3$ | $C_2H_5$ | $C_2H_5$ | O | 140/0.2; 1,5168 |

Verwendung der erfindungsgemäßen Wirkstoffe

In dem folgenden Beispiel werden die nachstehend angegebenen Verbindungen als Vergleichssubstanzen eingesetzt:

(A) = 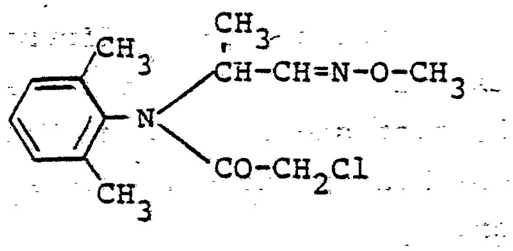 (bekannt aus DE-OS 28 47 827)

(B) = 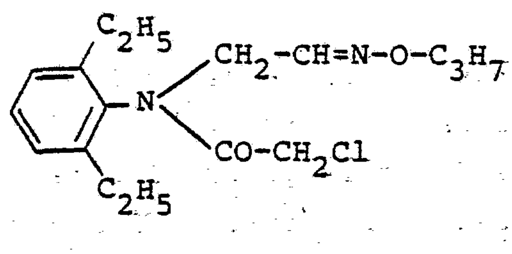 (bekannt aus DE-OS 28 47 827)

(C) = 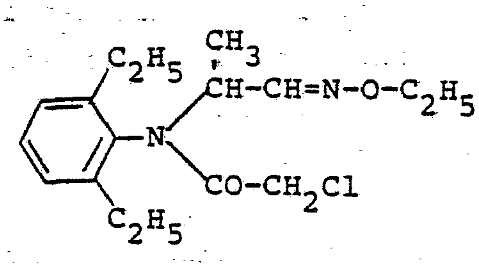 (bekannt aus DE-OS 28 47 827)

## Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die erfindungsgemäßen Wirkstoffe (1), (4) und (5) zeigen in diesem Test eine bessere herbizide Wirksamkeit als die bekannten Vergleichssubstanzen (A), (B) und (C).

## Patentansprüche

1. Hydroxamsäureester-Derivate der Formel

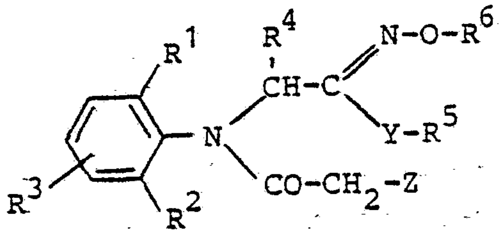

(I)

in welcher

Y für Sauerstoff oder Schwefel steht,

$R^1$ für Wasserstoff, Alkyl oder Alkoxy steht,

$R^2$ für Wasserstoff, Alkyl, Alkoxy oder Halogen steht,

$R^3$ für Wasserstoff, Alkyl, Alkoxy oder Halogen steht,

$R^4$ für Wasserstoff oder Alkyl steht,

$R^5$ für Alkyl, Alkenyl, Alkinyl oder Alkoxyalkyl steht,

$R^6$ für Wasserstoff, Alkyl, Alkenyl, Alkinyl oder Alkoxyalkyl steht oder

$R^4$ und $R^5$ gemeinsam für eine Alkylen-Kette stehen, oder

$R^5$ und $R^6$ gemeinsam für eine Alkylen-Kette stehen, sofern Y für Sauerstoff steht

und

Z für Halogen steht.

2. Hydroxamsäureester-Derivate der Formel (I), in denen Y für Sauerstoff oder Schwefel steht, $R^1$ für Wasserstoff, Alkyl mit 1 bis 4 Kohlenstoffatomen oder Alkoxy mit 1 bis 4 Kohlenstoffatomen steht, $R^2$ für Wasserstoff, Alkyl mit 1 bis 4 Kohlenstoffatomen, Alkoxy mit 1 bis 4 Kohlenstoffatomen, Fluor, Chlor oder Brom steht, $R^3$ für Wasserstoff, Alkyl mit 1 bis 4 Kohlenstoffatomen, Alkoxy mit 1 bis 4 Kohlenstoffatomen, Fluor, Chlor oder Brom steht, $R^4$ für Wasserstoff oder Alkyl mit 1 bis 4 Kohlenstoffatomen steht, $R^5$ für Alkyl mit 1 bis 4 Kohlenstoffatomen, Alkenyl mit bis zu 4 Kohlenstoffatomen, Alkinyl mit bis zu 4 Kohlenstoffatomen oder für Alkoxyalkyl mit 1 bis 4 Kohlenstoffatomen im Alkylteil und 1 bis 4 Kohlenstoffatomen im Alkoxyteil steht, $R^6$ für Wasserstoff, Alkyl mit 1 bis 4 Kohlenstoffatomen, Alkenyl mit bis zu 4 Kohlenstoffatomen, Alkinyl mit bis zu 4 Kohlenstoffatomen oder für Alkoxyalkyl mit 1 bis 4 Kohlenstoffatomen im Alkylteil und 1 bis 4 Kohlenstoffatomen im Alkoxyteil steht, und außerdem $R^4$ und $R^5$ auch gemeinsam für eine Alkylenkette mit 1 bis 3 Kohlenstoffatomen stehen, oder $R^5$ und $R^6$ gemeinsam für eine Alkylen-Kette mit 1 bis 3 Kohlenstoffatomen stehen, sofern Y für Sauerstoff steht, und ferner Z für Chlor oder Brom steht.

3. Verfahren zur Herstellung von Hydroxamsäureester-Derivaten der Formel

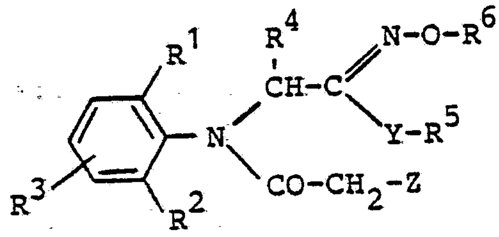

(I)

in welcher

Y für Sauerstoff oder Schwefel steht,

$R^1$ für Wasserstoff, Alkyl oder Alkoxy steht,

$R^2$ für Wasserstoff, Alkyl, Alkoxy oder Halogen steht,

$R^3$ für Wasserstoff, Alkyl, Alkoxy oder Halogen steht,

$R^4$ für Wasserstoff oder Alkyl steht,

$R^5$ für Alkyl, Alkenyl, Alkinyl oder Alkoxyalkyl steht,

$R^6$ für Wasserstoff, Alkyl, Alkenyl, Alkinyl oder Alkoxyalkyl steht oder

$R^4$ und $R^5$ gemeinsam für eine Alkylen-Kette stehen, oder

$R^5$ und $R^6$ gemeinsam für eine Alkylen-Kette stehen, sofern Y für Sauerstoff steht,

und

Z für Halogen steht,

dadurch gekennzeichnet, daß man N-Phenylglycin-Derivate der Formel

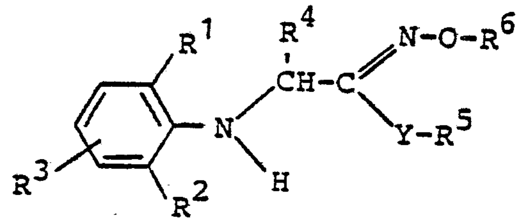

(II)

in welcher

$R^1$, $R^2$, $R^3$, $R^4$, $R^5$, $R^6$ und Y die obengenannte Bedeutung haben,

mit Halogenessigsäurechloriden oder -bromiden bzw. -anhydriden der Formeln

$$Z-CH_2-CO-Cl(Br) \qquad (IIIa)$$

bzw.

$$(Z-CH_2-CO)_2O \qquad (IIIb)$$

in welchen

Z die oben angegebene Bedeutung hat,

0051792

in Gegenwart eines Verdünnungsmittels und gegebenenfalls in Gegenwart eines Säurebindemittels umsetzt.

4. Herbizide Mittel, gekennzeichnet durch einen Gehalt an mindestens einem Hydroxamsäureester-Derivat der Formel (I).

5. Verfahren zur Bekämpfung von Unkräutern, dadurch gekennzeichnet, daß man Hydroxamsäureester-Derivate der Formel (I) auf Unkräuter und/oder deren Lebensraum ausbringt.

6. Verwendung von Hydroxamsäureester-Derivaten der Formel (I) zur Bekämpfung von Unkräutern.

7. Verfahren zur Herstellung von herbiziden Mitteln, dadurch gekennzeichnet, daß man Hydroxamsäureester-Derivate der Formel (I) mit Streckmitteln und/oder oberflächenaktiven Stoffen vermischt.

8. N-Phenylglycin-Derivate der Formel

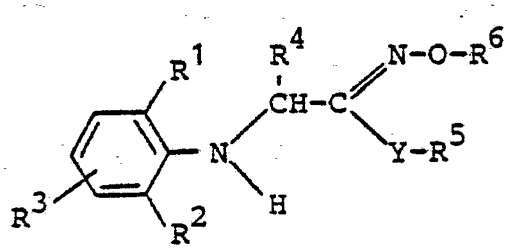

(II)

in welcher

Y für Sauerstoff oder Schwefel steht,

$R^1$ für Wasserstoff, Alkyl oder Alkoxy steht,

$R^2$ für Wasserstoff, Alkyl, Alkoxy oder Halogen steht,

$R^3$ für Wasserstoff, Alkyl, Alkoxy oder Halogen steht,

$R^4$ für Wasserstoff oder Alkyl steht,

$R^5$ für Alkyl, Alkenyl, Alkinyl oder Alkoxyalkyl steht,

$R^6$ für Wasserstoff, Alkyl, Alkenyl, Alkinyl oder Alkoxyalkyl steht oder

$R^4$ und $R^5$ gemeinsam für eine Alkylen-Kette stehen, oder

$R^5$ und $R^6$ gemeinsam für eine Alkylen-Kette stehen, sofern Y für Sauerstoff steht.

9. Verfahren zur Herstellung von N-Phenylglycin-Derivaten der Formel

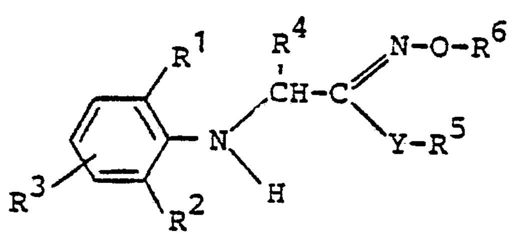

(II)

in welcher

Y für Sauerstoff oder Schwefel steht,

$R^1$ für Wasserstoff, Alkyl oder Alkoxy steht,

$R^2$ für Wasserstoff, Alkyl, Alkoxy oder Halogen steht,

$R^3$ für Wasserstoff, Alkyl, Alkoxy oder Halogen steht,

$R^4$ für Wasserstoff oder Alkyl steht,

$R^5$ für Alkyl, Alkenyl, Alkinyl oder Alkoxyalkyl steht,

$R^6$ für Wasserstoff, Alkyl, Alkenyl, Alkinyl oder Alkoxyalkyl steht oder

$R^4$ und $R^5$ gemeinsam für eine Alkylen-Kette stehen, oder

$R^5$ und $R^6$ gemeinsam für eine Alkylen-Kette stehen, sofern Y für Sauerstoff steht,

dadurch gekennzeichnet, daß man

a) Anilin-Derivate der Formel

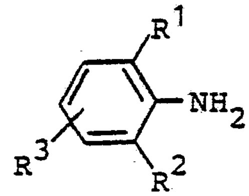

(IV)

in welcher

$R^1$, $R^2$ und $R^3$ die oben angegebene Bedeutung haben,

mit Hydroxamsäure-Derivaten der Formel

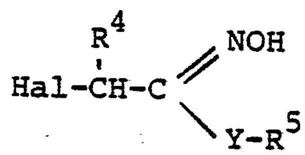

(V)

in welcher

$R^4$, $R^5$ und Y die oben angegebene Bedeutung haben und

Hal für Halogen steht,

in Gegenwart eines Verdünnungsmittels sowie gegebenenfalls in Gegenwart eines Säurebindemittels umsetzt und die dabei entstehenden N-Phenylglycin-Derivate der Formel

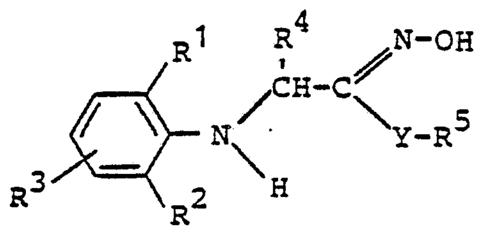

(IIa)

in welcher

$R^1$, $R^2$, $R^3$, $R^4$, $R^5$ und Y die oben angegebene Bedeutung haben,

gegebenenfalls anschließend mit Verbindungen der Formel

$$X-R^7 \quad (VI)$$

in welcher

$R^7$ für Alkyl, Alkenyl, Alkinyl oder Alkoxyalkyl steht und

X für Halogen oder ein Äquivalent eines Sulfat-Anions steht,

in Gegenwart eines Verdünnungsmittels sowie gegebenenfalls in Gegenwart eines Säurebindemittels umsetzt,

oder

b) Anilin-Derivate der Formel

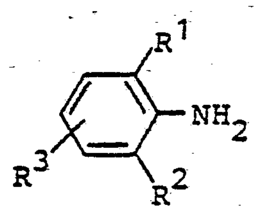

(IV)

in welcher

$R^1$, $R^2$ und $R^3$ die oben angegebene Bedeutung haben,

mit Hydroxamsäure-Derivaten der Formel

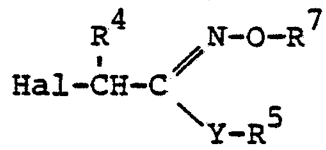

(VII)

in welcher

$R^4$, $R^5$, $R^7$, Hal und Y die oben angegebene Bedeutung haben,

in Gegenwart eines Verdünnungsmittels sowie gegebenenfalls in Gegenwart eines Säurebindemittels umsetzt,

oder

c) zur Herstellung derjenigen N-Phenylglycin-Derivate der Formel (II), in denen Y für Sauerstoff steht und $R^5$ und $R^6$ gemeinsam für Alkylen stehen, Anilin-Derivate der Formel

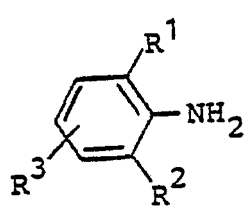

(IV)

in welcher

$R^1$, $R^2$ und $R^3$ die oben angegebene Bedeutung haben,

mit ringförmig verknüpften Alkylhydroxamsäureestern der Formel

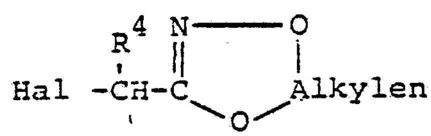

(VIII)

in welcher

$R^4$ und Hal die oben angegebene Bedeutung haben und

Alkylen für eine Alkylenkette steht,

in Gegenwart eines Verdünnungsmittels sowie gegebenenfalls in Gegenwart eines Säurebindemittels umsetzt.

Europäisches Patentamt

# EUROPÄISCHER RECHERCHENBERICHT

0051792

Nummer der Anmeldung

EP 81 10 8966.3

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | EP - A1 - 0 010 715 (BAYER)<br>* Ansprüche 1, 2, 3 * | 1,3,4 |
| A,D | & DE - A1 - 2 847 827 | |
| A,P | & DE - A1 - 2 931 103<br>-- | |
| A,P | EP - A2 - 0 019 858 (BAYER)<br>* Anpsrüche 1, 2 *<br>-- | 1,3 |
| A | DE - A1 - 2 709 854 (CIBA-GEIGY)<br>* Ansprüche 1, 16 *<br>-- | 1,4 |
| A | DE - A1 - 2 911 865 (CHEVRON RESEARCH)<br>* Ansprüche 1, 22 *<br>---- | 1,4 |

KLASSIFIKATION DER ANMELDUNG (Int. Cl.3)

C 07 C 131/105
C 07 C 83/10
C 07 C 119/18
C 07 C 149/24
C 07 D 273/00
A 01 N 37/22
A 01 N 37/36
A 01 N 43/88

RECHERCHIERTE SACHGEBIETE (Int. Cl.3)

A 01 N 37/22
A 01 N 37/24
A 01 N 37/36
A 01 N 43/88
C 07 C 83/10
C 07 C 119/16
C 07 C 119/18
C 07 C 131/105
C 07 C 149/24
C 07 D 273/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 26-01-1982 | KNAACK |